Europäisches Patentamt

European Patent Office

Office européen des brevets

(19)

(11) Veröffentlichungsnummer: **0 197 553 B1**

(12) **EUROPÄISCHE PATENTSCHRIFT**

(45) Veröffentlichungstag der Patentschrift: **24.07.91**

(51) Int. Cl.⁵: **A61M 1/28**

(21) Anmeldenummer: **86104833.8**

(22) Anmeldetag: **09.04.86**

(54) **Anordnung für die Peritonealdialyse sowie Konnektor hierfür.**

(30) Priorität: **12.04.85 DE 3513204**

(43) Veröffentlichungstag der Anmeldung:
**15.10.86 Patentblatt 86/42**

(45) Bekanntmachung des Hinweises auf die
Patenterteilung:
**24.07.91 Patentblatt 91/30**

(84) Benannte Vertragsstaaten:
**AT BE CH DE FR GB IT LI LU NL SE**

(56) Entgegenhaltungen:
**EP-A- 0 029 526**
**EP-A- 0 116 986**
**DE-A- 3 100 622**
**US-A- 4 306 976**

(73) Patentinhaber: **Fresenius AG**
**Gluckensteinweg 5**
**W-6380 Bad Homburg v.d.H.(DE)**

(72) Erfinder: **Mathieu, Bernd, Dr.**
**Galgenbergstr. 11**
**W-6683 Spiesen-Elversberg(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

## Beschreibung

Die Erfindung betrifft eine Anordnung für die Peritonealdialyse nach dem Oberbegriff des Patentanspruches 1, sowie einen Konnektor hierfür nach dem Oberbegriff des Patentanspruches 8.

Nierenkranke Patienten können bekanntlich im Endstadium ihrer Krankheit nur noch mit Hilfe der Hämodialyse oder der Peritonealdialyse am Leben erhalten werden. Dabei hat sich in jüngster Zeit wieder die Peritonealdialyse in den Vordergrund geschoben, da eine Methode entwickelt worden ist, die kontinuierlich und ambulant durchgeführt werden kann. Bei dieser sogenannten CAPD-Methode (continual ambulant peritoneal dialyses) wird im Gegensatz zur Hämodialyse, bei der der Patient im Abstand von zwei bis drei Tagen mit einem extrakorporalen Blutkreislauf an eine Hämodialysevorrichtung angeschlossen werden muß, dem Patienten ein Katheter in das Peritoneum implantiert, dessen Ende durch die Bauchwand ragt. Dieses Ende wird bei der Durchführung der Peritonealdialyse in gewissen Abständen mit einem Beutel verbunden, der eine sterile Dialysierflüssigkeit enthält.

Normalerweise durch die Niere abzutrennende Stoffwechselprodukte, wie Harnstoff u.dgl., werden durch die Dialysierflüssigkeit dialytisch über das Peritoneum entzogen, das somit als semipermeable Membran benutzt wird. Der Anschluß des Beutels mit der Dialysierflüssigkeit an den Katheter erfolgt über einen Konnektor, wobei ein weibliches Konnektorstück ständig mit dem Katheter verbunden bleibt und von dem Patienten getragen wird und ein männliches Konnektorstück, das in steriler Weise mit dem weiblichen Konnektorstück verbunden werden kann, am Ende eines Schlauchstücks eines mit der betreffenden Dialysierflüssigkeit gefüllten Beutels angeordnet ist.

Der Ablauf der verbrauchten Dialysierflüssigkeit aus dem Peritoneum erfolgt bei einem gattungsgemäßen Zweibeutel-System dadurch, daß zwischen dem männlichen Konnektorstück und dem gefüllten Beutel ein Y-Stück derart angeordnet ist, daß an dem freien Anschluß des Y-Stückes ein Leerbeutel angeschlossen werden kann. Zwischen dem weiblichen Konnektorstück und dem Baucheintritt, zwischen dem Y-Stück und dem Peritonealkatheter und zwischen dem Y-Stück und dem Leerbeutel ist je ein Absperrorgan, beispielsweise in Form einer Rollenklemme angeordnet. Durch entsprechendes Schließen und Öffnen dieser Rollenklemmen lassen sich somit die folgenden Fluidpassagen öffnen bzw. schließen: Vom vollen Beutel durch den Konnektor in das Peritoneum; vom vollen Beutel durch den Konnektor in den Leerbeutel; und vom Peritoneum in den Leerbeutel.

Absolute Sterilität ist eine der wichtigsten Grundvoraussetzungen bei der Durchführung einer CAPD, um Entzündungen durch eingebrachte Keime auszuschließen. Auf der Seite des männlichen Konnektorstückes, das über den Schlauch mit dem vollen Beutel verbunden ist, ergeben sich diesbezüglich keine Probleme, da das männliche Konnektorstück zusammen mit dem vollen Beutel in einer Einheit steril herstellbar und steril verpackbar ist, und nach einmaligem Gebrauch vernichtet wird bzw. zwischen zwei Anwendungen sterilisiert wird, wann das Schlauchstück vom Beutel getrennt werden kann. Das weibliche Konnektorstück jedoch, das bleibend mit dem Peritonealkatheter verbunden ist und daher von dem Patienten am Körper getragen werden muß, verlangt vor und nach jeder Dialyse eine gründliche Desinfektion, was beispielsweise dadurch erreicht wird, daß bei geschlossener Verbindung zwischen dem weiblichen Konnektorstück und dem Y-Stück das weibliche Konnektorstück mit einem geeigneten Desinfektionsmittel gefüllt oder eingesprüht und danach mit einem keimfrei schließenden Deckel verschlossen wird. Nicht immer sind diese Desinfektionsmittel physiologisch unbedenklich, so daß vor dem Dialysevorgang eine gründliche Spülung des weiblichen Konnektorstücks erforderlich ist, die beispielsweise dadurch erreicht wird, daß frische, sterile Dialysierflüssigkeit aus dem vollen Beutel bei geschlossener Verbindung zwischen dem Y-Stück und dem Peritonealkatheter zunächst über den Konnektor und das Y-Stück in den Leerbeutel geleitet wird, so daß das Desinfektionsmittel in dem weiblichen Konnektorstück zusammen mit etwaigen Partikeln, die sich beim Öffnen des vollen Beutels (beispielsweise durch Brechen eines Brechkonus) bilden können, in den Leerbeutel gespült werden.

Hierbei ergibt sich das Problem, daß in dem Anschluß des Y-Stückes, der zu dem Peritonealkatheter führt, ein gewisser Totraum verbleibt, über den die Dialysierflüssigkeit aus dem vollen Beutel in Richtung auf den leeren Beutel hinwegströmt, so daß Reste vom Desinfektionsmittel oder Partikel und eingeschleppte Keime in diesem nur schwer zu spülenden Bereich verbleiben können. Um somit eine zufriedenstellende Spülung des gesamten Systems sicherzustellen, ist es notwendig, relativ lange und mit reichlich Dialysierflüssigkeit zu spülen. Der gesamte Dialysevorgang wird somit unnötig verlängert und der Verbrauch an Dialysierflüssigkeit für reine Spülzwecke ist erheblich.

Eine derartige gattungsgemäße Vorrichtung ist aus der EP-A-29 526 bekannt, die einen einfachen, ungeschützten Konnektor aufweist, bei dem zwei mit den Fingern zu berührende Konnektorstücke zusammengesteckt werden. Diese Konnektorstücke weisen jeweils ein Anschlußstück für Schlauchstücke auf, wobei einer der beiden Konnektorstücke zusätzlich einen weiteren Anschluß aufweist, der somit y-förmig von der Haupt-Fluid-Passage abzweigt. Insofern unterscheidet sich diese Anordnung nicht von der vorstehend beschriebenen Anordnung, so daß in dem Schlauchsystem Toträume verbleiben bzw. Reste von Desinfektionsmittel mittel zurückbleiben, was für den Patienten nicht unerheblich ist.

Desweiteren sind aus dem Deutschen Gebrauchsmuster 78 34 790 und der EP-A-116 986 Konnektoren für die Peritonealdialyse bekannt, die jedoch jeweils nur einen Anschluß aufweisen, somit also im konnektierten Zustand keine Spülung oder Entfernung von Dialysierflüssigkeit zulassen.

Der Erfindung liegt daher die Aufgabe zugrunde, die eingangs erwähnte Anordnung sowie den Konnektor gemäß Oberbegriff des Anspruchs 8 so fortzubilden, daß ein steriles Konnektieren und ein Freispülen der gesamten Schlauchanordnung und des Konnektors von Verunreinigungen möglich sind.

Die Lösung dieser Aufgabe erfolgt durch die kennzeichnenden Merkmale des Anspruches 1 bzw. 8.

Gemäß Anspruch 1 wird auf das Y-Stück mit all seinen Nachteilen dadurch verzichtet, daß ein dritter Anschluß für den Leerbeutel zur Aufnahme der Spülflüssigkeit bzw. der verbrauchten Dialysierflüssigkeit am männlichen Konnektorstück getrennt von dem Anschluß für die frische Dialysierflüssigkeit angeordnet ist.

Vorteilhafterweise weist das weibliche Konnektorstück ein Verschlußorgan auf, welches normalerweise die Fluidpassage von dem vollen Beutel in das Peritoneum verschließt. Durch dieses Verschlußorgan wird somit in der ersten Phase des Konnektierens des männlichen und des weiblichen Konnektorstückes ein geschlossener Raum geschaffen, der eine Zufluß (vom vollen Beutel) und einen Abfluß (zu dem Leerbeutel) aufweist und der teilweise mit dem Desinfektionsmittel gefüllt ist, das sich in dem weiblichen Konnektorstück oberhalb des Verschlußorgans befindet. In dieser ersten Phase öffnet das zentrale Rohrstück des männlichen Konnektorteils das Verschlußorgan noch nicht. Ein Öffnen des vollen Beutels, beispielsweise durch Brechen eines Brechkonus, einer Verschlußeinrichtung in der Verbindungsleitung, bewirkt nun, daß frische Dialysierflüssigkeit aus dem vollen Beutel in den geschlossenen Raum durch den ersten Anschluß eintritt. Desinfektionsmittel und Partikel in diesem geschlossenen Raum werden nun von der einströmenden frischen Dialysierflüssigkeit durch den dritten Anschluß am männlichen Konnektorstück in den Leerbeutel gespült. Da dieser abgeschlossene Raum allseitig von ebenen Flächen begrenzt ist, verbleiben keine Toträume, in welchen sich Reste des Desinfektionsmittels bzw. Keime lange halten können, so daß die Entfernung des Desinfektionsmittels und Spülung dieses Raumes mit einem Minimum an Zeitaufwand und Spüllösung bewerkstelligt wird.

Um die Ausbildung von Toträumen in dem Konnektor zu verhindern, weist das weibliche Konnektorstück vorteilhafterweise im Bereich seiner Einführöffnung, d.h. in dem Bereich, der in das männliche Konnektorstück eingeführt wird, ein Abdichtteil auf, das in unmittelbarer Nachbarschaft zur Einführöffnung angeordnet ist, so daß der geschlossene Raum, der sich beim Ineinanderführen des weiblichen und des männlichen Konnektorstückes bildet, im wesentlichen frei von Hinterschneidungen ist.

Die jeweiligen Unteransprüche haben vorteilhafte Weiterbildungen der Erfindung zum Inhalt.

Weitere Einzelheiten, Merkmale und vorteile der vorliegenden Erfindung ergeben sich aus der nachfolgenden Beschreibung einer Ausführungsform anhand der Zeichnung.

Es zeigt:

Fig. 1    in schematischer Schnittdarstellung die wesentlichen Komponenten des erfindungsgemäßen Zweibeutel-Systems;

Fig. 2    eine erste Konnektierphase des weiblichen Konnektorstückes und des männlichen Konnektorstückes, in welcher der Spülvorgang stattfindet;

Fig. 3    eine zweite Konnektierphase des weiblichen Konnektorstückes und des männlichen Konnektorstückes, in welcher die verbrauchte Dialysierflüssigkeit aus dem Peritonealraum in den Leerbeutel abfließt; und

Fig. 4    die gleiche Konnektierphase wie in Fig. 3, wobei jedoch in dieser Stellung frische Dialysierflüssigkeit aus dem vollen Beutel in den Peritonealraum einfließt.

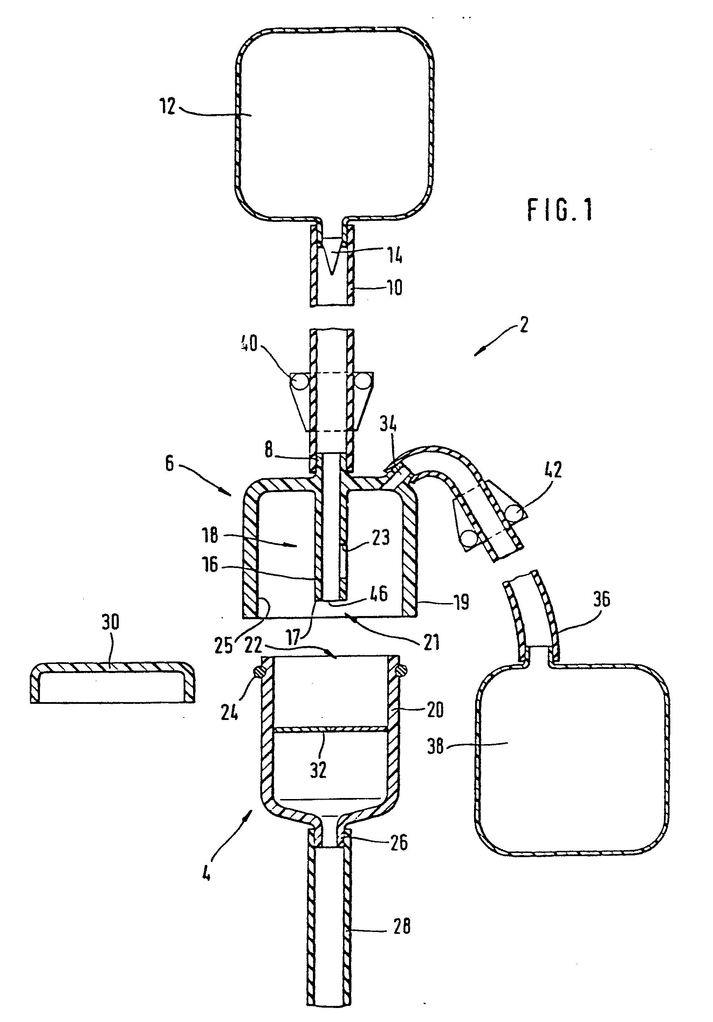

Wie aus Fig. 1 hervorgeht, weist ein Zweibeutel-System 2 im wesentlichen ein weibliches Konnektorstück 4 und ein männliches Konnektorstück 6 auf, die in Fig. 1 voneinander getrennt dargestellt sind. Das männliche Konnektorstück 6 ist als einseitig verschlossenes zylindrisches Bauteil ausgeführt und weist an seinem verschlossenen Ende einen ersten Anschluß 8 für eine Zufuhrleitung 10 auf, welche mit einem Beutel 12 mit frischer Dialysierflüssigkeit verbunden ist. In der Zufuhrleitung 10 ist im Bereich des Beutels 12 ein zu öffnendes Verschlußteil 14 vorgesehen, das den Beutel gegenüber der Zufuhrleitung abschließt und im Bedarfsfall geöffnet werden kann, beispielsweise dadurch, daß ein Abbrechteil hiervon abgebrochen wird.

Wie aus der Zeichnung hervorgeht, erstreckt sich der erste Anschluß 8 von der Außenseite des männlichen Konnektorstückes 6 koaxial zu dem männlichen Konnektorstück 6 in Form eines innenliegenden Rohrstückes 16 in einen Aufnahmebereich 18, der zwischen dem Rohrstück 16 und einer dieses Rohrstück 16 um- und übergreifenden Schutzhülse 19 vorgesehen ist. Dabei ist der Frontbereich 17 des Rohrstückes 16 so weit vom Randbereich der Schutzhülse entfernt, daß keine Berührungsmöglichkeit mehr besteht. Weiterhin ist vorteilhafterweise benachbart zum Frontbereich 17 des Rohrstückes 16 wenigstens eine radiale Öffnung 23 vorgesehen. Der Anschluß der Zufuhrleitung 10 an den ersten Anschluß 8 kann in üblicher Weise durch Aufschrumpfen, Verkleben, eine Luer-Verbindung, eine Klemme oder dgl. erfolgen.

Gemäß Fig. 1 weist das weibliche Konnektorstück 4 einen hohlzylindrischen Einführabschnitt 20 mit einer Einführöffnung 22 auf. Der Außendurchmesser des Einführabschnittes 20 ist so bemessen, daß sich das weibliche Konnektorstück 4 mit leichtem Spiel in den Aufnahmebereich 18 des männlichen Konnektorstückes 6 einführen läßt. Um im konnektierten Zustand eine flüssigkeitsdichte Verbindung zwischen dem weiblichen Konnektorstück 4 und dem männlichen Konnektorstück 6 zu erhalten, ist unmittelbar benachbart zu der Einführöffnung 22 des weiblichen Konnektorstückes 4 ein ringförmiges Abdichtteil 24 angeordnet, das beispielsweise als O-Ring ausgebildet ist. An der der Einführöffnung 22 gegenüberliegenden Seite des weiblichen Konnektorstückes 4 ist ein zweiter Anschluß 26 ausgebildet, mit dem das weibliche Konnektorstück 4 über eine Zufuhrleitung 28 mit einem in der Zeichnung nicht dargestellten Peritonealkatheter verbindbar ist. Mit einer in Fig. 1 mit 30 bezeichneten abnehmbaren Kappe wird die Einführöffnung 22 des weiblichen Konnektorstückes 4 verschlossen, wenn keine Peritonealdialyse vorgenommen wird. Wie aus Fig. 1 ersichtlich, weist also das weibliche Konnektorteil 4 eine hohlzylindrische Form mit im wesentlichen kreisförmigem Querschnitt auf, wobei das eine Ende die Einführöffnung 22 aufweist und das andere Ende zum Schlauchanschluß 26 verjüngt ist.

Des weiteren kann sich die radiale Öffnung 23 auch vom Frontbereich 17 her unter Ausbildung von ein oder mehreren Schlitzen im Rohrstück 16 zum Anschluß 8 unter Beibehaltung der Funktion erstrecken.

In dem weiblichen Konnektorstück 4 ist zwischen dessen Einführöffnung 22 und dem zweiten Anschluß 26 ein Verschlußorgan 32 angeordnet, welches sich bei ausreichender mechanischer Belastung in Richtung auf den zweiten Anschluß 26 hin öffnet und einen Fluiddurchlaß von der Einführöffnung 22 zu dem zweiten Anschluß 26 freigibt. Dieses Verschlußorgan 32 kann beispielsweise eine Ventilplatte mit einem sternförmigen Schlitz sein, wie sie beispielsweise in der DE-OS 3 210 148 der Anmelderin beschrieben ist.

Das männliche Konnektorstück 6 weist einen dritten Anschluß 34 vorteilhafterweise im Bereich des ersten Anschlusses 8 auf, an welchem eine Zufuhrleitung 36 zu einem Leerbeutel 38 anschließbar ist bzw. angeschlossen ist. Der Leerbeutel 38 dient zur Aufnahme der verbrauchten Dialysierflüssigkeit aus dem Peritonealraum bzw. zur Aufnahme der Spülflüssigkeit aus dem vollen Beutel 12. Im Nahbereich des männlichen Konnektorstücks 6 ist sowohl auf der Zufuhrleitung 10 als auch auf der Zufuhrleitung 36 je ein Absperrorgan 40 bzw. 42 angeordnet. Diese Absperrorgane 40 und 42 sind beispielsweise in Form von Rollen- oder Quetschklemmen ausgeführt und dienen dazu, den Fluiddurchlaß durch die Leitung 10 bzw. durch die Leitung 36 zu öffnen bzw. zu sperren.

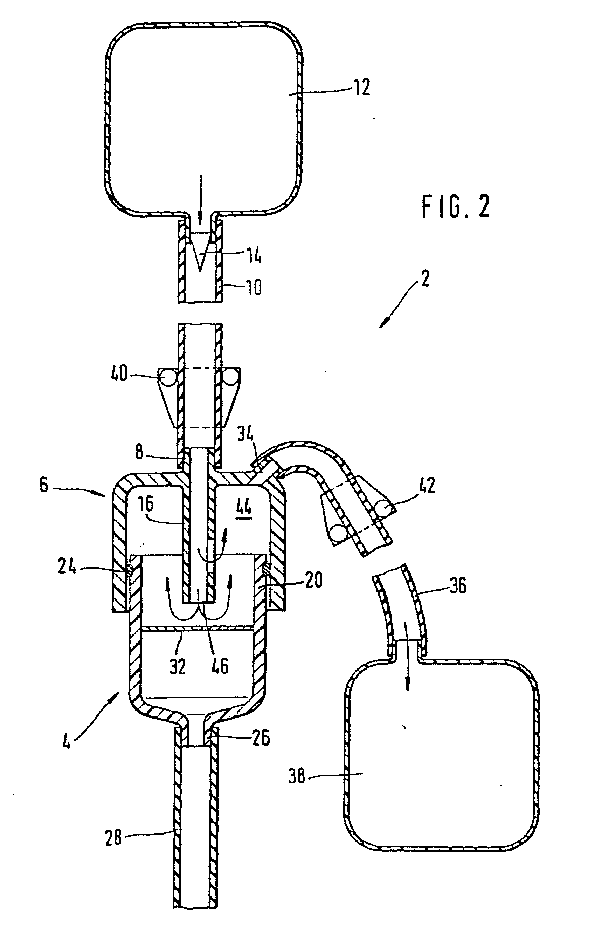

Im folgenden soll nun, insbesondere unter Bezugnahme auf die Fig. 2 bis 4, die Anwendung und Wirkungsweise des vorliegenden Zweibeutel-Systems beschrieben werden:

Wenn sich ein Patient der Peritonealdialyse unterziehen muß, wird die Kappe 30 von dem weiblichen Konnektorstück 4 abgenommen, so daß die Einführöffnung 22 des weiblichen Konnektorstückes 4 freiliegt. Der Bereich oberhalb des Verschlußorgans 32 ist normalerweise mit einem Desinfektionsmittel gefüllt oder eingesprüht, das vor dem Einbringen von frischer Dialysierflüssigkeit aus dem vollen Beutel 12 in den Leerbeutel 38 gespült werden soll. Hierzu wird nach Abnehmen der Kappe 30 von dem weiblichen Konnektorstück 4 das weibliche Konnektorstück 4 in eine Aufnahmeöffnung 21 des männlichen Konnektorstückes 6 derart eingeführt, daß ein erster Konnektionszustand gem. Fig. 2 erreicht wird, in welchem der O-Ring 24 des weiblichen Konnektorstückes 4 an der inneren Umfangswand 25 der Schutzhülse 19 des männlichen Konnektorstückes 6 abdichtend anliegt, so daß ein abgeschlossener Raum 44 entsteht, der von der Innenwandung des männlichen Konnektorstückes 6, dem O-Ring 24 und dem Verschlußorgan 32 begrenzt wird. Das Einführen des weiblichen Konnektorstückes 4 in das männliche Konnektorstück 6 bis zu diesem ersten Konnektionszustand gem. Fig. 2 kann beispielsweise durch eine in der Zeichnung nicht dargestellte Schraubverbindung oder dgl. unterstützt und gesichert werden. Festzuhalten ist, daß in der ersten Konnektionsphase der Frontbereich 17 des Rohrstückes 16 nicht das Verschlußorgan 32 öffnet.

Somit erstreckt sich in dem ersten Konnektionszustand gem. Fig. 2 das Innenrohr 16 des männlichen Konnektorstückes 6 in die Einführöffnung 22 des weiblichen Konnektorstückes 4 derart, daß eine Mündungsöffnung 46 im Rohrstück 16 innerhalb der Einführöffnung 22 und oberhalb des Verschlußorgans 32 angeordnet ist.

Dieser erste Konnektionszustand gem. Fig. 2 wird als Spülstellung bezeichnet.

Vor dem Abführen der verbrauchten Dialysierflüssigkeit aus dem Peritonealraum in den Leerbeutel 38 und vor dem Zuführen von frischer Dialysierflüssigkeit in den Peritonealraum aus dem vollen Beutel 12 wird ein Spülvorgang durchgeführt, insbesondere, um das Desinfektionsmittel aus dem abgeschlossenen Raum 44 zu entfernen. Hierzu werden die beiden Rollenklemmen 40 und 42 an den Zufuhrleitungen 10 und 36 geöffnet, so daß eine freie Fluidpassage von dem Verschlußorgan 14 am vollen Beutel 12 zu dem Leerbeutel 38 vorliegt. Danach wird der volle Beutel 12 durch Abbrechen des Abbrechkonusteils vom Verschlußorgan 14 geöffnet und frische Dialysierflüssigkeit strömt aus dem Beutel 12 durch die Zufuhrleitung 10 und das Innenrohr 16 zur radialen Öffnung 23 und zur Mündungsöffnung 46 des Rohrstücks 16 und durch den abgeschlossenen Raum 44, den dritten Anschluß 34 und die Zufuhrleitung 36 in den Leerbeutel 38, wie in Fig. 2 durch die Strömungspfeile dargestellt.

Das Desinfektionsmittel in dem abgeschlossenen Raum 44 und beim Öffnen des Verschlußteils 14 entstehende Partikel werden somit durch die frische Dialysierflüssigkeit aus dem vollen Beutel 12 aus dem abgeschlossenen Raum 44 in den Leerbeutel 38 gespült. Die Effektivität des Spülvorganges wird dadurch erhöht, daß einerseits die Mündungsöffnung 46 des Rohrstücks 16 nahe über dem Verschlußorgan 32 angeordnet ist, so daß die Spüllösung den abgeschlossenen Raum 44 vollständig ausfüllt und das Desinfektionsmittel durch den dritten Anschluß 34 in den Leerbeutel 38 verdrängt. Andererseits ist der offene Durchmesser des Rohrstückes 16 bzw. der Mündungsöffnugn 46 vorzugsweise größer als der offene Durchmesser des dritten Anschlusses 34, so daß eine Druckspülung des abgeschlossenen Raumes 44 durch die frische Dialysierflüssigkeit aus dem vollen Beutel 12 erfolgt.

Durch das Anordnen des O-Ringes 24 im unmittelbaren Nahbereich der Einführöffnung 22 des weiblichen Konnektorstückes 4 entsteht in dem abgeschlossenen Raum 44 im wesentlichen kein Totraum und keine Hinterschneidung, in welchem bzw. in welcher sich das Desinfektionsmittel halten kann, so daß eine gründliche und vor allem eine schnelle Spülung des abgeschlossenen Raumes 44 gewährleitstet ist.

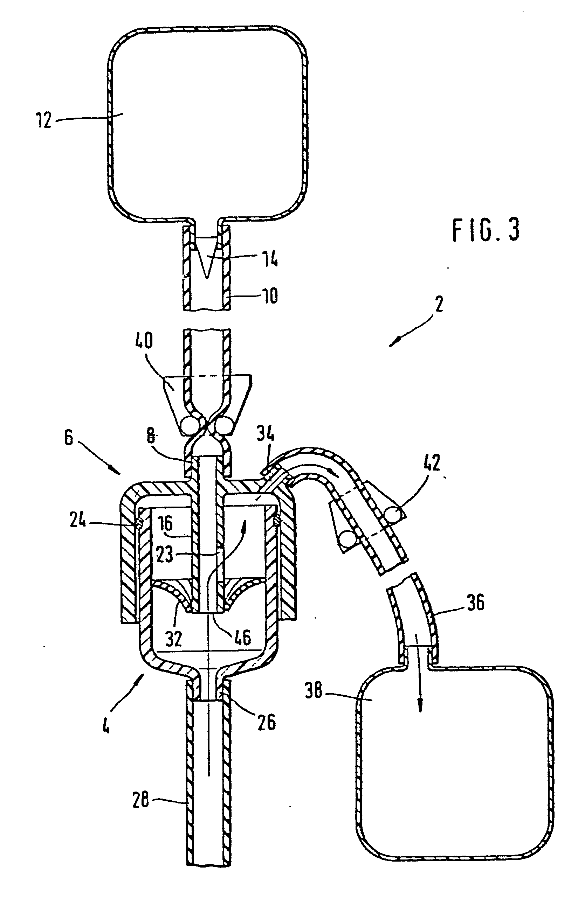

Nach dem Beendigen des Spülvorganges erfolgt das Ablassen der verbrauchten Dialysierflüssigkeit aus dem Peritonealraum des Patienten. Hierzu wird zunächst die Rollenklemme 40 in der Zufuhrleitung 10 derart betätigt, daß der Zulauf von frischem Dialysat aus dem vollen Beutel 12 in den abgeschlossenen Raum 44 unterbrochen wird. Die Rollenklemme 42 der Zufuhrleitung 36 zu dem Leerbeutel 38 bleibt weiterhin geöffnet. Danach wird das weibliche Konnektorstück 4 durch Einschrauben oder -schieben weiter in den Aufnahmebereich 18 des männlichen Konnektorstücks 6 eingeführt, bis ein zweiter Konnektionszustand gem. Fig. 3 erreicht ist.

Wie aus Fig. 3 hervorgeht, öffnet der Frontbereich 17 des Rohrstückes 16 das Verschlußorgan 32, wobei letzteres in Richtung auf den zweiten Anschluß 26 des weiblichen Konnektorstückes 4 gedrängt wird. Die radiale Öffnung 23 im Rohrstück 16 liegt dabei vorteilhafterweise so weit von dem Frontbereich 17 entfernt, daß keine die Fluidpassage hemmende Belegung durch das Verschlußorgan 32 durch den Anschluß 34 möglich ist. Insofern wird also eine Strömungsverbindung von der Schlauchleitung 28 durch den Anschluß 26, das geöffnete Verschlußorgan 32, die Mündungsöffnung 46 und die radiale Öffnung 23 des Rohrstückes 16 zur Schlauchleitung 36 und den Leerbeutel 38 bzw. Anschluß 8 hergestellt. In dieser Fluidpassage strömt nun die verbrauchte Dialysierflüssigkeit aus dem Peritonealraum des Patienten durch den Konnektor 2 in den Leerbeutel 38, wie in Fig. 3 durch die Strömungspfeile dargestellt, wobei das Verschlußelement 40 geschlossen bleibt.

Dieser zweite Konnektionszustand des weiblichen Konnektorstückes 4 und des männlichen Konnektorstückes 6 wird als Ein/Ablaßstellung bezeichnet.

Nachdem die verbrauchte Dialysierflüssigkeit aus dem Peritonealraum des Patienten vollständig abgelassen worden ist, wird die Rollenklemme 42 der Zufuhrleitung 36 zu dem nunmehr mit der verbrauchten Dialysierflüssigkeit gefüllten Leerbeutel 38 geschlossen, so daß die Verbindung zwischen dem Peritonealraum und dem Leerbeutel 38 unterbrochen ist.

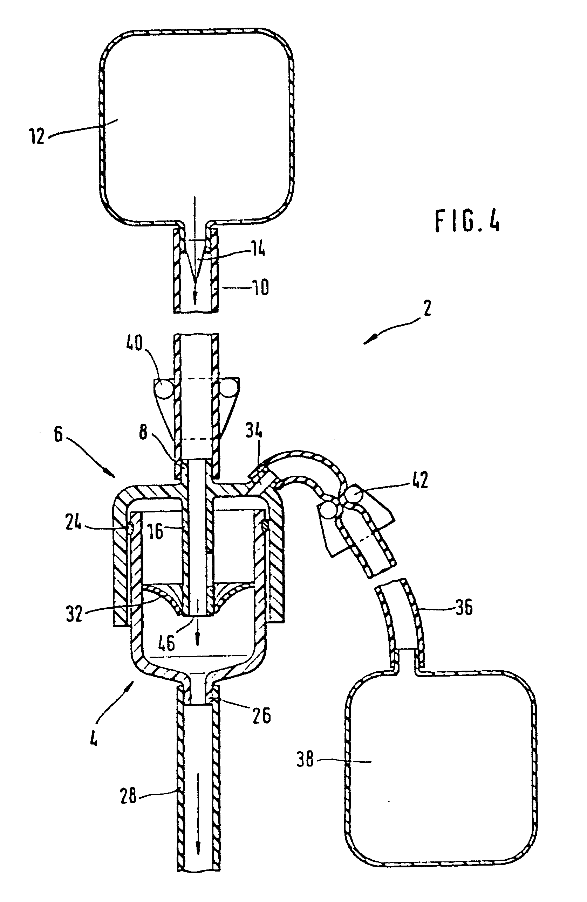

Daraufhin wird gem. Fig. 4 die Rollenklemme 40 der Zufuhrleitung 10 geöffnet, so daß eine Fluidpassage von dem vollen Beutel 12 durch den männlichen Konnektor 6 und dem weiblichen Konnektor 4 zu dem Pertonealraum des Patienten geöffnet wird. Die Stellung des weiblichen Konnektors 4 innerhalb des männlichen Konnektors 6 entspricht hierbei weiterhin dem zweiten Konnektionszustand gem. Fig. 3, d.h. das Verschlußorgan 32 ist in Richtung auf den zweiten Anschluß 26 unter Deformierung geöffnet, so daß die Mündungsöffnung 46 des Innenrohres 16 mit der Schlauchleitung 28 in Strömungsverbindung steht. Somit kann die frische Dialysierflüssigkeit aus dem vollen Beutel 2 ungehindert in den Peritonealraum des Patienten einströmen, wie in Fig. 4 durch die Strömungspfeile angedeutet.

Nachdem der Inhalt des vollen Beutels 12 in den Peritonealraum des Patienten geflossen ist, erfolgt die Diskonnektion des männlichen Konnektorstückes 6 von dem weiblichen Konnektorstück 4, wobei zunächst wieder der erste Konnektionszustand gem. Fig. 2 erreicht wird, in welchem das Verschlußorgan 32 wieder elastisch in seine Ausgangsstellung zurückgekehrt ist, so daß der Zugang zu dem Peritonealraum des Patienten wieder verschlossen ist. Danach erfolgt die endgültige Diskonnektierung des weiblichen Konnektorstückes 4 von dem männlichen Konnektorstück 6. In den Freiraum oberhalb des Verschlußorgans 32 wird ein geeignetes Desinfektionsmittel eingefüllt oder eingesprüht, wonach die Einführöffnung 22 des weiblichen Konnektorstückes 4 wieder mit der desinfizierten bzw. sterilen Kappe 30 verschlossen wird.

Vorteilhafterweise werden der volle Beutel 12, die Zufuhrleitung 10, das männliche Konnektorstück 6, die Zufuhrleitung 36 und der Leerbeutel 38 als Einheit steril hergestellt und verpackt, so daß sich bezüglich der Keimfreiheit der männlichen Konnektorseite keine Probleme ergeben. Eine solche Einheit ist vorteilhafterweise mit einer evakuierten Schutzhülle in Form eines sterilen Schutzbeutels umgeben, wie dies in der europäischen Patentschrift 50 255 beschrieben ist. Nach dem Gebrauch dieser Einheit, d.h. nach Beendigung der Dialyse, werden das männliche Konnektorstück 6 sowie die daran angeschlossenen Beutel 12 und 38 weggeworfen bzw. vernichtet.

In einer weiteren Ausführungsform kann das zentrale Rohrstück 16 des männlichen Konnektorteils 6 auch ohne radiale Öffnung 23 versehen sein. In diesem Fall öffnet sich das Verschlußorgan 32 unter Ausbildung einer ersten Strömungsverbindung, die, wie vorstehend erwähnt, durch das zentrale Rohrstück 16 unmittelbar führt, und einer zweiten Strömungsverbindung, die an diesem Rohrstück 16 vorbei durch sich bildende Schlitze u.dgl. in dem Verschlußorgan 32 in den Raum 44 führt.

Die Auswahl der Materialien zur Herstellung der beiden Konnektorstücke und der entsprechenden Zufuhrleitungen liegt im Bereich des fachmännischen Handelns, es kommen beispielsweise physiologisch unbedenkliche Kunststoffe oder Metalle in Frage.

## Patentansprüche

1. Anordnung für Peritonealdialyse mit einem Konnektor, der ein erstes Konnektorstück (4) mit einer Einführöffnung(22) und ein zweites Konnektorstück (6) mit einem zentralem Rohrstück (16) aufweist, das in die Einführöffnung (22) des ersten Konnektorstücks (4) einführbar ist, mit einem ersten Anschluß (8) an einem der beiden Konnektorstücke zur Verbindung mit einem ersten Schlauchstück (10), das mit einem Dialysierflüssigkeit aufweisenden ersten Beutel (12) verbunden ist, mit einem zweiten Anschluß (26) an dem anderen Konnektorstück zur Verbindung mit einem zweiten Schlauchstück (28), das mit einem Peritonealkatheter verbindbar ist, und mit einem dritten Anschluß (34) an einem der beiden Konnektorstücke zur Verbindung mit einem dritten Schlauchstück (36) und mit einem zweiten Beutel (38) sowie mit wenigstens zwei Klemmen (40,42) zum Abklemmen des ersten und dritten Schlauchstücks (10,36), **dadurch gekennzeichnet, daß** das zentrale Rohrstück (16) des zweiten Konnektorstücks (6) von einer Außenhülse (19) umfaßt und übergriffen ist, daß das erste Konnektorstück (4) auf seiner Außenoberfläche von einer ringförmigen Dichtung (24) umfaßt ist, die mit der Innenfläche (25) der Außenhülse (19) eine den Innenraum des Konnektors abdichtende Anordnung bildet, und daß der dritte Anschluß (34) von der Außenhülse (19) abgeht.

2. Anordnung nach Anspruch 1, **dadurch gekennzeichnet**, daß das weibliche Konnektorstück (4) ein Verschlußorgan (32) zwischen seiner Einführöffnung (22) und dem zweiten Anschluß(26) aufweist.

3. Anordnung nach Anspruch 1, **durch gekennzeichnet**, daß der Frontbereich (17) des Rohrstücks (16) axial näher zur Aufnahmeöffnung (21) des männlichen Konnektorstücks (6) angeordnet ist als der dritte Anschluß (34).

4. Anordnung nach Anspruch 1 **dadurch gekennzeichnet**, daß das zentrale Rohrstück (16) wenigstens eine radiale Öffnung (23) aufweist, die eine Strömungsverbindung bei der Kupplung der beiden Konnektorteile (4,6) zwischen dem zweiten Anschluß (26) und dem dritten Anschluß (34) erzeugt.

5. Anordnung nach Anspruch 1, **dadurch gekennzeichnet**, daß das weibliche Konnektorstück (4) benachbart zu der Einführöffnung (22) auf seiner Außenoberfläche eine ringförmige Dichtung (24) aufweist, die bei Konnektion der beiden Konnektorteile (4, 6) eine flüssigkeitsdichte Keimsperre darstellt.

6. Anordnung nach Anspruch 1, **dadurch gekennzeichnet**, daß die Einführöffnung (22) des weiblichen Konnektorstücks (4) durch eine abnehmbare Kappe (30) verschließbar ist.,

7. Anordnung nach Anspruch 1, **dadurch ge-**

kennzeichnet, daß der erste Anschluß (8) des männlichen Konnektorstücks (6) über eine Schlauchleitung (10) mit einem mit frischer Dialysierflüssigkeit gefüllten ersten Beutel (12) verbunden ist, wobei die Schlauchleitung (10) mit einer ersten Rollenklemme (40) absperrbar ist, daß der dritte Anschluß (34) des männlichen Konnektorstücks (6) über die zweite Schlauchleitung (36) mit dem Leerbeutel (38) verbunden ist, wobei die zweite Schlauchleitung (36) mit einer zweiten Rollenklemme (42) absperrbar ist, und daß die gesamte Anordnung steril in einem evakuierten Umbeutel vorgesehen ist.

8. Männliches Kupplungsstück bestehend aus dem zweiten Kupplungsstück von Anspruch 1, insbesondere für ein Zweibeutel-System (2) nach dem Oberbegriff des Anspruchs 1 mit einem zentralen Rohrstück (16), das von einer Außenhülse (19) umfaßt und übergriffen ist und das mit einem auf der Außenhülse angeordneten ersten Anschluß (8) in Strömungsverbindung steht, **dadurch gekennzeichnet**, daß auf der Außenhülse (19) ein weiterer Anschluß (34) zur Verbindung mit einem zweiten Beutel (38) vorgesehen ist.

9. Männliches Kupplungsstück nach Anspruch 8, **dadurch gekennzeichnet**, daß das zentrale Rohrstück (16) wenigstens eine radiale Öffnung (23) aufweist.

10. Männliches Kupplungsstück nach Anspruch 9, **dadurch gekennzeichnet**, daß der dritte Anschluß (34) weiter von der Einführröffnung (21) abliegt als der Frontbereich (17) des zentralen Rohrstücks (16).

## Claims

1. Arrangement for peritoneal dialysis comprising a connector having a first connector piece (4) with an introduction opening (22) and a second connector piece (6) with a central tube section (16) which is introduceable into the introduction opening (22) of the first connector piece (4), further comprising a first connection (8) on one of the two connector pieces for connection to a first tube portion (10) which is connected to a first bag (12) containing dialysis solution, a second connection (26) on the other connector piece for connection to a second tube portion (28) which is connectable to a peritoneal catheter, and a third connection (34) on one of the two connector pieces for connection to a third tube portion (36) and a second bag (38) and at least two clamps (40, 42) for clamping off the first and third tube portion (10, 36), characterized in that the central tube section (16) of said second connector piece (6) is surrounded and engaged by an outer sleeve (19), that the first connector piece (4) is surrounded on its outer surface by an annular sealing (24) which with the inner face (25) of the outer sleeve (19) forms an arrangement sealing the interior of the connector, and that the third connection (34) originates from the outer sleeve (19).

2. Arrangement according to claim 1, characterized in that the female connector piece (4) comprises a closure member (32) between its introduction opening (22) and the second connection (26).

3. Arrangement according to claim 1, characterized in that the front region (17) of the tube section (16) is disposed axially closer to the receiving opening (21) of the male connector piece (6) than the third connection (34).

4. Arrangement according to claim 1, characterized in that the central tube section (16) comprises at least one radial opening (23) which when the two connector pieces (4, 6) are coupled establishes a flow connection between the second connection (26) and the third connection (34).

5. Arrangement according to claim 1, characterized in that the female connector piece (4) comprises adjacent the introduction opening (22) on its outer surface an annular sealing (24) which on connection of the two connector pieces (4, 6) represents a fluid-tight germ barrier.

6. Arrangement according to claim 1, characterized in that the introduction opening (22) of the female connector piece (4) is sealable by a removable cap (30).

7. Arrangement according to claim 1, characterized in that the first connection (8) of the male connector piece (6) is connected via a flexible tube (19) to a first bag (12) filled with fresh dialysis solution, the flexible tube (10) being closable with a first roller clamp (40), that the third connection (34) of the male connector piece (6) is connected via the second flexible tube (36) to the empty bag (38), the second flexible tube (36) being closable with a second roller clamp (42), and that the entire arrangement is disposed sterile in an evacuated envelope.

8.  Male coupling piece consisting of the second coupling piece of claim 1, in particular for a two-bag system (2) according to the preamble of claim 1, comprising a central tube section (16) which is surrounded and engaged by an outer sleeve (19) and which is in flow connection with a first connection (8) disposed on the outer sleeve, characterized in that on the outer sleeve (19) a further connection (34) is provided for connection to a second bag (38).

9.  Male coupling piece according to claim 8, characterized in that the central tube section (16) comprises at least one radial opening (23).

10. Male coupling piece accoding to claim 9, characterized in that the third connection (34) lies further from the introduction opening (21) than the front region (17) of the central tube section (16).

**Revendications**

1.  Système pour dialyse péritonéale comprenant un raccord qui comporte une première pièce de raccord (4) pourvue d'un orifice d'entrée (22) et une deuxième pièce de raccord (6) pourvue d'un bout de tube central (16) qui peut être introduit dans l'orifice d'entrée (22) de la première pièce de raccord (4), une première tubulure (8) prévue sur l'une des deux pièces de raccord, en vue d'un raccordement à un premier tuyau flexible (10) qui est raccordé à une première poche (12) contenant un liquide de dialyse, une deuxième tubulure (26) prévue sur l'autre pièce de raccord en vue d'un raccordement à un deuxième tuyau flexible (28) qui peut être raccordé à un cathéter péritonéal, et une troisième tubulure (34) prévue sur l'une des deux pièces de raccord en vue d'un raccordement à un troisième tuyau flexible (36) et à une deuxième poche (38), ainsi qu'au moins deux pinces (40, 42) pour obturer par pinçage le premier et le troisième tuyau flexible (10, 36), caractérisé en ce que le bout de tube central (16) de la deuxième pièce de raccord (6) est entouré et recouvert d'un manchon extérieur (19), que la première pièce de raccord (4) est entourée, au niveau de sa surface extérieure, d'un joint d'étanchéité annulaire (24), qui forme, avec la surface intérieure (25) du manchon extérieur (19), un montage assurant l'étanchéité de l'intérieur (25) du raccord et que la troisième tubulure (34) part du manchon extérieur (19).

2.  Système suivant la revendication 1, caractérisé en ce que la pièce de raccord femelle (4) présente un organe d'obturation (32) entre son orifice d'entrée (22) et la deuxième tubulure (26).

3.  Système suivant la revendication 1, caractérisé en ce que la zone frontale (17) du bout de tube (16) est disposée, dans le sens axial, plus près de l'orifice de réception (21) de la pièce de raccord mâle (6) que de la troisième tubulure (34).

4.  Système suivant la revendication 1, caractérisé en ce que le bout de tube central (16) présente au moins une lumière radiale (23), qui assure, lors de l'assemblage des deux pièces de raccord (4, 6), une communication d'écoulement entre la deuxième tubulure (26) et la troisième tubulure (34).

5.  Système suivant la revendication 1, caractérisé en ce que la pièce de raccord femelle (4) présente, sur sa surface extérieure, à proximité de son orifice d'entrée (22), un joint d'étanchéité annulaire (24), qui, lors de l'assemblage des deux pièces de raccord (4, 6), forme une barrière stérile étanche.

6.  Système suivant la revendication 1, caractérisé en ce que l'orifice d'entrée (22) de la pièce de raccord femelle (4) peut être obturé par une coiffe amovible (30).

7.  Système suivant la revendication 1, caractérisé en ce que la première tubulure (8) de la pièce de raccord mâle (6) est raccordée, par un tuyau flexible (10), à une première poche (12) remplie de liquide de dialyse frais, le tuyau flexible (10) pouvant être obturé par une première pince à galets (40), que la troisième tubulure (34) de la pièce de raccord mâle (6) est raccordée à la poche vide (38) par le deuxième tuyau flexible (36), ce deuxième tuyau flexible (36) pouvant être obturé par une deuxième pince à galets (42), et que l'ensemble du système est logé de manière stérile dans une poche sous vide.

8.  Pièce de raccord mâle constituée de la deuxième pièce de raccord suivant la revendication 1, en particulier pour un système à deux poches (2), suivant le préambule de la revendication 1, pourvue d'un bout de tube central (16) qui est entouré et recouvert avec dépassement par un manchon extérieur (19) et qui est en communication d'écoulement avec une première tubulure (8) prévue sur le manchon extérieur, caractérisée en ce que, sur le manchon extérieur (19) est prévue une tubulure supplémentaire (34) destinée au raccordement à une deuxième poche (38).

9. Pièce de raccord mâle suivant la revendication 8, caractérisée en ce que le bout de tube central (16) présente au moins une lumière radiale (23).

10. Pièce de raccord mâle suivant la revendication 9, caractérisée en ce que la troisième tubulure (34) est disposée plus loin de l'orifice d'entrée (21) que de la zone frontale (17) du bout de tube central (16).

FIG.1

FIG. 2

FIG. 3

FIG.4